(19) Europäisches Patentamt
European Patent Office
Office européen des brevets

(11) Veröffentlichungsnummer: **0 004 917**
**B1**

(12) **EUROPÄISCHE PATENTSCHRIFT**

(45) Veröffentlichungstag der Patentschrift: **13.05.81**

(51) Int. Cl.³: **C 07 D 249/08, A 01 N 43/64**

(21) Anmeldenummer: **79101049.9**

(22) Anmeldetag: **06.04.79**

# (54) Oximino-triazolyl-äthane, Verfahren zu ihrer Herstellung sowie ihre Verwendung als Fungizide.

(30) Priorität: **18.04.78 DE 2816817**

(43) Veröffentlichungstag der Anmeldung: **31.10.79 Patentblatt 79/22**

(45) Bekanntmachung des Hinweises auf die Patenterteilung: **13.05.81 Patentblatt 81/19**

(84) Benannte Vertragsstaaten: **BE CH DE FR GB IT NL SE**

(56) Entgegenhaltungen:
**DE - A - 2 635 883**
**DE - A - 2 704 682**
**DE - A - 2 723 942**

(73) Patentinhaber: **BAYER AG**
**Zentralbereich Patente, Marken und Lizenzen**
**D-5090 Leverkusen 1, Bayerwerk (DE)**

(72) Erfinder: **Krämer, Wolfgang, Dr.**
**Am Eckbusch 39/162**
**D-5600 Wuppertal 1 (DE)**
Erfinder: **Büchel, Karl Heinz, Prof. Dr.**
**Bergerheide 62**
**D-5600 Wuppertal 1 (DE)**
Erfinder: **Timmler, Helmut, Dr.**
**Tersteegenweg 16**
**D-5600 Wuppertal 1 (DE)**
Erfinder: **Brandes, Wilhelm, Dr.**
**Eichendorffstrasse 3**
**D-5650 Leichlingen 1 (DE)**
Erfinder: **Frohberger, Paul-Ernst, Dr.**
**Willi-Baumeister-Strasse 5**
**D-5090 Leverkusen 1 (DE)**

EP 0 004 917 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Aktive Korrosionsschutzpigmente auf Calciumoxid-, Aluminiumoxid- und Eisenoxidbasis, Verfahren zu ihrer Herstellung und ihre Verwendung

Die vorliegende Erfindung betrifft neue Oximinotriazolyl-äthane, ein Verfahren zu ihrer Herstellung sowie ihre Verwendung als fungizide.

Es ist bereits bekannt geworden, daß Imidazolyl-oximäther und deren Salze, wie beispielsweise 1 - (2,4 - Dichlorphenyl) - 2 - (imidazol - 1 - yl) - 0 - (2,4 - dichlorbenzyl) - äthanonoxim - nitrat, fungizide Eigenschaften aufweisen (vgl. DE—OS 26 57 578); weiterhin ist die fungizide Wirkung von Triazolyl-alkanonen und -alkanolen bekannt, so z.B. vom 1 - [4 - (4' - Chlorphenoxy) - phenyl] - 2 - (1,2,4 - triazol - 1 - yl) - äthan - 1 - on (vgl. DE—OS 24 31 407). Die Wirkung der vorbekannten Verbindungen ist jedoch, insbesondere bei niedrigen Aufwandmengen und -konzentrationen, nicht immer befriedigend.

Es wurden neue Oximino-triazolyl-äthane der allgemeinen Formel

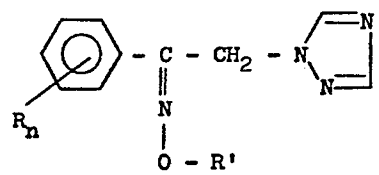

(I)

in welcher

R für Halogen, Nitro und Cyano, für Alkyl und Alkylsulfonyl mit jeweils 1 bis 4 Kohlenstoffatomen, für Alkoxy und Alkylthio mit 1 bis 2 Kohlenstoffatomen, für Halogenalkyl mit bis zu 4 Kohlenstoffund bis zu 5 Halogenatomen, ferner für Phenyl oder Phenoxy steht, wobei letztere beiden Reste durch Halogen, Cyano, Nitro, Halogenalkyl mit bis zu 2 Kohlenstoff- und bis zu 3 Halogenatomen substituiert sein können,

R' für Alkyl, Alkenyl und Alkinyl mit jeweils bis zu 4 Kohlenstoffatomen steht, sowie für Benzyl oder Styryl steht, wobei die beiden letztgenannten Reste durch Halogen, Cyano, Nitro, Amino, Alkyl mit bis zu 4 Kohlenstoffatomen, Phenyl, Phenoxy und Halogenalkyl mit bis zu 2 Kohlenstoff- und bis zu 3 Halogenatomen substituiert sein können, und

n für ganze Zahlen von 0 bis 3 steht,

und deren physiologisch verträglichen Säureadditions- Salze und Metallsalz-Komplexe gefunden. Sie weisen starke fungizide Eigenschaften auf.

Die Verbindungen der Formel (I) können in der syn- oder anti-Form vorliegen; vorwiegend fallen sie als Gemische beider Formen an.

Man erhält die Oximino-triazolyl-äthane der Formel (I), wenn man die Salze der Oxime der Formel

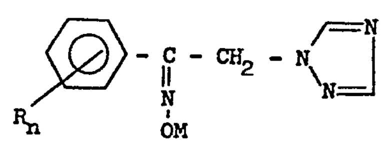

(II)

in welcher

R und n die oben angegebene Bedeutung haben und

M für ein Alkalimetall, eine quarternäre Ammonium- oder Phosphonium-Gruppe steht,

mit einem Halogenid der Formel

R'—Hal (III)

in welcher

R' die oben angegebene Bedeutung hat und

Hal für Chlor oder Brom steht,

in Gegenwart eines Verdünnungsmittels umsetzt.

Weiterhin können die erfindungsgemäß erhältlichen Oximinotriazolyl-äthane der Formel (I) durch Umsetzung mit Säuren in die Salze überführt werden, bzw. durch Reaktion mit Metallsalzen die entsprechenden Metallkomplexe erhalten werden.

Ueberaschenderweise zeigen die erfindungsgemäßen Oximinotriazolyl-äthane eine erheblich höhere fungizide Wirksamkeit, insbesondere gegen Mehltau- und Rostarten, als die aus dem Stand der Technik bekannten Imidazolyl-oximäther, wie beispielsweise 1-(2,4-Dichlorphenyl)-2-(imidazol-1-yl)-0-(2,4-dichlorbenzyl)-äthanonoxim, welche chemisch und wirkungsmäßig naheliegende Stoffe sind. Die erfindungsgemäßen Wirkstoffe stellen somit eine Bereicherung der Technik dar.

Die erfindungsgemäßen Oximino-triazolyl-äthane sind durch die Formel (I) allgemein definiert.

Bevorzugt sind diejenigen Oximino-triazolyl-äthane der Formel (I), in denen *R* für Chlor, Brom, Phenyl, Chlorphenyl, Bromphenyl, Nitrophenyl, Phenoxy, Chlorphenoxy, Bromphenoxy oder Nitrophenoxy steht; *n* für die Zahlen 1 oder 2 steht und *R'* für Methyl, Aethyl, n-Propyl, Isopropyl, n-Butyl, Isobutyl, sek.-Butyl oder tert.-Butyl; Allyl, 2-Methylallyl; Propargyl; Benzyl, das gegebenenfalls ein- oder zweifach substituiert ist durch Chlor, Brom, Nitro, Methyl, Aethyl, Phenyl oder Phenoxy; sowie für gegebenenfalls ein- oder zweifach durch Chlor substituiertes Styryl steht.

Im Einzelnen seien außer den Herstellungsbeispielen und den Beispielen der Tabelle 1 die folgenden Verbindungen genannt:

1-(2,4-Dichlorphenyl)-1-(allyloximino)-2-(1,2,4-triazol-1-yl)-äthan
1-(2,4-Dichlorphenyl)-1-(2,4-dichlorbenzyloximino)-2-(1,2,4-triazol-1-yl)-äthan
1-(2,4-Dichlorphenyl)-1-(propargyloximino)-2-(1,2,4-triazol-1-yl)-äthan
1-(4-Chlorphenyl)-1-(2,4-dichlorbenzyloximino)-2-(1,2,4-triazol-1-yl)-äthan
1-(4-Chlorphenyl)-1-(4-chlorbenzyloximino)-2-(1,2,4-triazol-1-yl)-äthan
1-(2,4-Dichlorphenyl)-1-(2,6-dichlorbenzyloximino)-2-(1,2,4-triazol-1-yl)-äthan
1-(4-Chlorphenyl)-1-(2,6-dichlorbenzyloximino)-2-(1,2,4-triazol-1-yl)-äthan
1-(4-Chlorphenyl)-1-(allyloximino)-2-(1,2,4-triazol-1-yl)-äthan
1-(4-Phenoxyphenyl)-1-(allyloximino)-2-(1,2,4-triazol-1-yl)-äthan
1-(4,4'-Chlorphenoxyphenyl)-1-(allyloximino)-2-(1,2,4-triazol-1-yl)-äthan
1-(4,4'-Chlorphenoxyphenyl)-1-(2,4-dichlorbenzyloximino)-2-(1,2,4-triazol-1-yl)-äthan
1-(2,4-Dichlorphenyl)-1-(4-nitrobenzyloximino)-2-(1,2,4-triazol-1-yl)-äthan
1-(2,4-Dichlorphenyl)-1-(4-aminobenzyloximino)-2-(1,2,4-triazol-1-yl)-äthan
1-(4-Phenoxyphenyl)-1-(2,4-dichlorbenzyloximino)-2-(1,2,4-triazol-1-yl)-äthan
1-(2,4-Dichlorphenyl)-1-(methyloximino)-2-(1,2,4-triazol-1-yl)-äthan
1-(4-Bromphenyl)-1-(allyloximino)-2-(1,2,4-triazol-1-yl)-äthan
1-(4,4'-Chlorbiphenylyl)-1-(2,4-dichlorbenzyloximino)-2-(1,2,4-triazol-1-yl)-äthan
1-(4-Phenoxyphenyl)-1-(4-chlorbenzyloximino)-2-(1,2,4-triazol-1-yl)-äthan
1-(4-Phenoxyphenyl)-1-(2,6-dichlorbenzyloximino)-2-(1,2,4-triazol-1-yl)-äthan
1-(4,4'-Chlorphenoxyphenyl)-1-(methyloximino)-2-(1,2,4-triazol-1-yl)-äthan
1-(4-Phenoxyphenyl)-1-(propargyloximino)-2-(1,2,4-triazol-1-yl)-äthan
1-(4-Bromphenyl-1-(2,4-dichlorbenzyloximino)-2-(1,2,4-triazol-1-yl)-äthan
1-(4,4'-Bromphenoxyphenyl)-1-(2,4-dichlorbenzyloximino)-2-(1,2,4-triazol-1-yl)-äthan
1-(4,4'-Bromphenoxyphenyl)-1-(allyloximino)-2-(1,2,4-triazol-1-yl)-äthan
1-(4,4'-Bromphenoxyphenyl)-1-(4-chlorbenzyloximino)-2-(1,2,4-triazol-1-yl)-äthan
1-(2,4-Dichlorphenyl)-1-(2-chlorbenzyloximino)-2-(1,2,4-triazol-1-yl)-äthan
1-(2,4-Dichlorphenyl)-1-(butyloximino)-2-(1,2,4-triazol-1-yl)-äthan
1-(2,4-Dichlorphenyl)-1-(3,4-dichlorbenzyloximino)-2-(1,2,4-triazol-1-yl)-äthan
1-(2,4-Dichlorphenyl)-1-(3-nitrobenzyloximino)-2-(1,2,4-triazol-1-yl)-äthan
1-(4-Bromphenyl)-1-(2,6-dichlorbenzyloximino)-2-(1,2,4-triazol-1-yl)-äthan
1-(4,4'-Bromphenoxyphenyl)-1-(3,6-dichlorbenzyloximino)-2-(1,2,4-triazol-1-yl)-äthan
1-(4-Bromphenyl)-1-(4-chlorbenzyloximino)-2-(1,2,4-triazol-1-yl)-äthan
1-(4,4'-Chlorbiphenylyl)-1-(2,6-dichlorbenzyloximino)-2-(1,2,4-triazol-1-yl)-äthan
1-(2,4-Dichlorphenyl)-1-(2-methylallyloximino)-2-(1,2,4-triazol-1-yl)-äthan
1-(4,4'-Chlorbiphenylyl)-1-(4-chlorbenzyloximino)-2-(1,2,4-triazol-1-yl)-äthan
1-(4,4'-Chlorphenoxyphenyl)-1-(4-chlorbenzyloximino)-2-(1,2,4-triazol-1-yl)-äthan
1-(4,4'-Chlorphenoxyphenyl)-1-(2,6-dichlorbenzyloximino)-2-(1,2,4-triazol-1-yl)-äthan
1-(4,4'-Chlorphenoxyphenyl)-1-(2-methylallyloximino)-2-(1,2,4-triazol-1-yl)-äthan
1-(4,4'-Chlorphenoxyphenyl)-1-(propargyloximino)-2-(1,2,4-triazol-1-yl)-äthan
1-(4,4'-Chlorphenoxyphenyl)-1-(2-chlorbenzyloximino)-2-(1,2,4-triazol-1-yl)-äthan
1-(4,4'-Chlorphenoxyphenyl)-1-(3,4-dichlorbenzyloximino)-2-(1,2,4-triazol-1-yl)-äthan
1-(4,4'-Chlorphenoxyphenyl)-1-(3-nitrobenzyloximino)-2-(1,2,4-triazol-1-yl)-äthan
1-(4,4'-Nitrophenoxyphenyl)-1-(allyloximino)-2-(1,2,4-triazol-1-yl)-äthan
1-(2,4-Dichlorphenyl)-1-(methylbenzyloximino)-2-(1,2,4-triazol-1-yl)-äthan
1-(2,4-Dichlorphenyl)-1-(3-phenoxybenzyloximino)-2-(1,2,4-triazol-1-yl)-äthan
1-(4,4'-Chlorphenoxyphenyl)-1-(3-phenoxybenzyloximino)-2-(1,2,4-triazol-1-yl)-äthan
1-(4,4'-Chlorphenoxyphenyl)-1-(4-methylbenzylamino)-2-(1,2,4-triazol-1-yl)-äthan
1-(2,4-Dichlorphenyl)-1-(styryloximino)-2-(1,2,4-triazol-1-yl)-äthan
1-(4-Biphenylyl)-1-(styryloximino)-2-(1,2,4-triazol-1-yl)-äthan
1-(4,4'-Chlorbiphenylyl)-1-(styryloximino)-2-(1,2,4-triazol-1-yl)-äthan
1-(4-Phenoxyphenyl)-1-(styryloximino)-2-(1,2,4-triazol-1-yl)-äthan
1-(4,4'-Chlorphenoxyphenyl)-1-(styryloximino)-2-(1,2,4-triazol-1-yl)-äthan
1-(2,4-Dichlorphenyl)-1-(2,4-dichlorstyryloximino)-2-(1,2,4-triazol-1-yl)-äthan
1-(4,4'-Chlorbiphenylyl)-1-(2,4-dichlorstyryloximino)-2-(1,2,4-triazol-1-yl)-äthan
1-(4,4'-Chlorphenoxyphenyl)-1-(2,4-dichlorstyryloximino)-2-(1,2,4-triazol-1-yl)-äthan
1-(3,4-Dichlorphenyl)-1-(2,4-dichlorbenzyloximino)-2-(1,2,4-triazol-1-yl)-äthan

1-(3,4-Dichlorphenyl)-1-(butyoximino)-2-(1,2,4-triazol-1-yl)-äthan
1-(3,4-Dichlorphenyl)-1-(allyloximino)-2-(1,2,4-triazol-1-yl)-äthan

Verwendet man beispielsweise das Natriumalkanolat des 1 - (2,4 - Dichlorphenyl) - 1 - oximino - 2 - (1,2,4 - triazol - 1 - yl) - äthan und 4 - Chlorbenzylchlorid als Ausgangsstoffe, so kann der Reaktionsablauf durch das folgende Formelschema wiedergegeben werden:

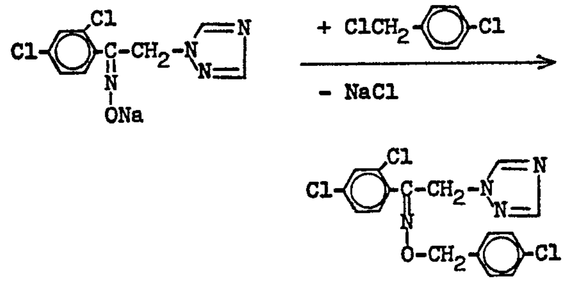


Die als Ausgangsstoffe zu verwendenden Oximino-äthylate sind durch die Allgemeine Formel (II) definiert. *M* steht vorzugsweise für die Alkalimetalle Lithium, Natrium und Kalium. *M* steht außerdem vorzugsweise für folgende quarternäre Ammoniumgruppen:

Tetrabutylammonium, N-Benzyl-N,N,N-trimethylammonium, Hexadecyl-trimethylammonium, 2-Hydroxyäthyl-trimethylammonium, Tetraäthylammonium, Tetramethylammonium, Tetra-n-propylammonium, (Cyclopropylmethyl)-trimethylammonium, Methyl-trioctylammonium, N-Phenyl-N,N,N-trimethyl-ammonium, N-(4-Methylbenzyl)-N,N,N-trimethylammonium, N-Benzyl-N,N-dimethyl-N-dodecyl-ammonium, N,N-Dibenzyl-N,N dimethyl-ammonium, Benzyldimethyl-n-hexadecylammonium, Benzyldimethyl-tetradecylammonium, Benzyl-tributyl-ammonium, Benzyl-triäthyl-ammonium, Butyl-tripropyl-ammonium, Octadecyl-trimethyl-ammonium, Tetrahexylammonium, Tetraoctyl-ammonium, Tetra-pentyl-ammonium, Tricaprylmethyl-ammonium und Hexadecylpyridinium sowie vorzugsweise für folgende Phosphoniumgruppen: Tetraphenylphosphonium, Hexadecyltributyl-phosphonium, Aethyltriphenyl-phosphonium oder Methyl-triphenyl-phosphonium.

Die Oximino-äthylate der Formel (II) sind noch nicht bekannt. Sie lassen sich jedoch in allgemein bekannter Art und Weise herstellen, indem man die entsprechenden Oxime mit geeigneten starken Basen, wie Alkalimetallamiden oder -hydriden, quarternären Ammoniumhydroxiden oder Phosphoniumhydroxiden in einem indifferenten Lösungsmittel umsetzt. Die Oxime, die zur Herstellung der Oximinoäthylate der Formel (II) als Ausgangsstoffe dienen, sind ebenfalls noch nicht bekannt. Sie werden erhalten, indem man Aethanone der Formel

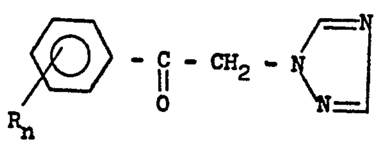

(IV)

in welcher

R und n die oben angegebene Bedeutung haben, mit Hydroxylamin in Gegenwart eines Lösungsmittels, vorzugsweise Alkohole bzw. wäßrige Alkohole, bei Temperaturen zwischen 20 und 100°C, vorzugsweise zwischen 50 und 80°C, umsetzt. Dabei wird das Hydroxylamin vorzugsweise in Form seiner Salze, insbesondere als Hydrochlorid, gegebenenfalls in Gegenwart eines Säurebindemittels, wie beispielsweise Natriumacetat, eingesetzt. Die Isolierung erfolgt dadurch, daß man das während der Umsetzung entstandene Produkt gegebenenfalls nach Abdestillieren des Lösungsmittels nach üblichen Methoden aufarbeitet (vergleiche auch Herstellungsbeispiele).

Die als Ausgangsstoffe verwendeten Aethanone der Formel (IV) sind bekannt (vergleiche DT—OS 24 31 407 [LeA 15 735]) bzw. werden nach den dort beschriebenen Verfahren erhalten, z.B. durch Umsetzung entsprechender Halogenketone mit 1,2,4-Triazolen in Gegenwart eines Säurebinders (vergleiche auch Herstellungsbeispiele). Die dazu notwendigen Halogenketone sind bekannt (vergleiche Bulletin de la Societé Chimique de France *1955*, Seiten 1363—1383). Die noch nicht bekannten Stoffe lassen sich nach den dort beschriebenen Verfahren herstellen (man vergleiche hierzu auch die Angaben in der US—Patentschrift 3 679 697 und der DT—OS 20 63 857).

Als beispiele für die Oxime, die den erfindungsgemäß als Ausgangsstoffe zu verwendenden Oximino-äthylaten der Formel (II) zugrunde liegen, seien genannt:

1-(4-Chlorphenyl)-1-(oximino-2-(1,2,4-triazol-1-yl)-äthan
1-(2,4-Dichlorphenyl)-1-oximino-2-(1,2,4-triazol-1-yl)-äthan
1-(3,4-Dichlorphenyl)-1-oximino-2-(1,2,4-triazol-1-yl)-äthan
1-(4-Bromphenyl)-1-oximino-2-(1,2,4-triazol-1-yl)-äthan
1-(4-Nitrophenyl)-1-oximino-2-(1,2,4-triazol-1-yl)-äthan
1-(4-Cyanphenyl)-1-oximino-2-(1,2,4-triazol-1-yl)-äthan

1-(2-Methylphenyl)-1-oximino-2-(1,2,4-triazol-1-yl)-äthan
1-(2-Methoxyphenyl)-1-oximino-2-(1,2,4-triazol-1-yl)-äthan
1-(2-Aethyl-4-chlorphenyl)-1-oximino-2-(1,2,4-triazol-1-yl)-äthan
1-(2-Aethylthiophenyl)-1-oximino-2-(1,2,4-triazol-1-yl)-äthan
1-(4-Methylsulfonylphenyl)-1-oximino-2-(1,2,4-triazol-1-yl)-äthan
1-(2,4,5-Trichlorphenyl)-1-oximino-2-(1,2,4-triazol-1-yl)-äthan
1-(4-Biphenylyl)-1-oximino-2-(1,2,4-triazol-1-yl)-äthan
1-(4,4'-Chlorbiphenylyl)-1-oximino-2-(1,2,4-triazol-1-yl)-äthan
1-(4-4'-Brombiphenylyl)-1-oximino-2-(1,2,4-triazol-1-yl)-äthan
1-(4-4'-Nitrobiphenylyl)-1-oximino-2-(1,2,4-triazol-1-yl)-äthan
1-(4-Phenoxyphenyl)-1-oximino-2-(1,2,4-triazol-1-yl)-äthan
1-(4-4'-Chlorphenoxyphenyl)-1-oximino-2-(1,2,4-triazol-1-yl)-äthan
1-(4-4'-Bromphenoxyphenyl)-1-oximino-2-(1,2,4-triazol-1-yl)-äthan
1-(4-4'-Nitrophenoxyphenyl)-1-oximino-2-(1,2,4-triazol-1-yl)-äthan

Die weiterhin als Ausgangsstoffe zu verwendenden Halogenide sind durch die Formel (III) allgemein definiert.

Die Halogenide der Formel (III) sind allgemein bekannte Verbindungen der organischen Chemie. Als Beispiele seien genannt: Methylchlorid und -bromid, Aethylchlorid und -bromid, n-Propylchlorid und -bromid, Isopropylchlorid und -bromid, n-Butylchlorid und -bromid, tert.-Butylchlorid und -bromid, Allylchlorid und -bromid, 2-Methylallylchlorid und -bromid, 4-Chlorbenzylchlorid und -bromid, 2,4-Dichlorbenzylchlorid und -bromid, 2-Chlorbenzylchlorid und -bromid, 3,4-Dichlorbenzylchlorid und -bromid, 3-Nitrobenzylchlorid und -bromid, 4-Methylbenzylchlorid und -bromid, 3-Phenoxybenzylchlorid und -bromid, 4-Phenoxybenzylchlorid und -bromid, 4-Phenylbenzylchlorid und -bromid, Styrylchlorid und -bromid, 2,4-Dichlorstyrylchlorid und -bromid, Propargylchlorid und -bromid.

Zur Herstellung von Säureadditionssalzen der Verbindungen der Formel (I) kommen alle physiologisch verträglichen Säuren infrage. Hierzu gehören vorzugsweise die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z.B. p-Toluolsulfonsäure und 1,5-Naphthalindisulfonsäure.

Die Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von metallsalz-Komplexen der Verbindungen der Formel (I) kommen vorzugsweise Salze von Metallen der II, bis IV.Haupt- und der I, und II, sowie IV. bis VIII. Nebengruppe infrage, wobei Kupfer, Zink, Mangan, Magnesium, Zinn, Eisen und Nickel beispielhaft genannt seien. Als Anionen der Salze kommen solche in Betracht, die sich von physiologischen Säuren ableiten. Hierzu gehören vorzugsweise die Halogenwasserstoffsäuren, wie z.B. die Chlorwasserstoffsäure und die Bromwasserstoffsäure, ferner Phosphorsäure, Salpetersäure und Schwefelsäure. Die Metallsalzkomplexe der Verbindungen der Formel (I) können in einfacher Weise nach üblichen Verfahren erhalten werden, so z.B. durch Lösen des Metallsalzes in Alkohol, z.B. Aethanol, und Hinzufügen zur Verbindung der Formel (I). Man kann Metallsalzkomplexe in bekannter Weise, z.B. durch Abfiltrieren, isolieren und gegebenenfalls durch Umkristallisation reinigen.

Für die erfindungsgemäße Umsetzung kommen als Verdünnungsmittel inerte organische Lösungsmittel infrage. Hierzu gehören vorzugsweise Aether, wie Diäthyläther und Dioxan; aromatische Kohlenwasserstoffe, wie Toluol und Benzol; in einzelnen Fällen auch chlorierte Kohlenwasserstoffe, wie Chloroform, Methylenchlorid oder Tetrachlorkohlenstoff; sowie Hexamethylphosphorsäure-triamid.

Die Reaktionstemperaturen können in einem größeren Bereich variiert werden. Im allgemeinen arbeitet man zwischen 20 und 150°C, vorzugsweise bei Raumtemperatur. In einzelnen Fällen ist es vorteilhaft bei der Siedetemperatur des Lösungsmittels, beispielsweise zwischen 60 und 100°C zu arbeiten.

Bei der Durchführung des erfindungsgemäßen Verfahrens setzt man auf 1 Mol des Oximino-äthylats der Formel (II) vorzugsweise 1 bis 3 Mol Halogenid der Formel (III) ein. Zur Isolierung der Endprodukte wird das Reaktionsgemisch vom Lösungsmittel befreit und der Rückstand mit Wasser und einem organischen Lösungsmittel versetzt. Die organische Phase wird abgetrennt, in üblicher Weise aufgearbeitet und gereinigt, gegebenenfalls wird das Salz hergestellt. In einer bevorzugten Ausführungsform wird zweckmäßigerweise so verfahren, daß man von einem 1 - Phenyl - 1 - oximino - 2 - (1,2,4 - triazol - 1 - yl) - äthan - Derivat ausgeht, letzteres in einem geeigneten inerten organischen Lösungsmittel mittels Alkalimetall-hydrid oder Alkalimetall-amid in das Oximino-äthylat der Formel (II) überführt, und letzteres ohne Isolierung sofort mit einem Halogenid der Formel (III) umsetzt, wobei unter Austritt von Alkalihalogenic die erfindungsgemäßen Verbindungen der Formel (I) in einem Arbeitsgang erhalten werden.

Nach einer weiteren bevorzugten Ausführungsform werden zweckmäßigerweise die Herstellung der Oximino-äthylate der Formel (II) sowie die erfindungsgemäße Umsetzung in einem Zweiphasensystem, wie beispielsweise wässrige Natron- oder Kalilauge/Toluol oder Methylenchlorid, unter Zusatz von 0.01—1 Mol eines Phasen-Transfer-Katalysators, wie beispielsweise Ammonium- oder Phosphoniumverbindungen, durchgeführt, wobei in der organischen Phase oder an der Grenzfläche die Aethylate entstehen und mit den in der organischen Phase befindlichen Halogeniden umgesetzt werden.

Die erfindungsgemäßen Wirkstoffe weisen eine starke fungitoxische Wirkung auf. Sie schädigen Kulturpflanzen in den zur Bekämpfung von Pilzen notwendigen Konzentrationen nicht. Aus diesen Gründen sind sie für den Gebrauch als Pflanzenschutzmittel zur Bekämpfung von Pilzen geeignet. Fungitoxische Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die erfindungsgemäßen Wirkstoffe haben ein breites Wirkungsspektrum und können angewandt werden gegen parasitäre pilze, die oberirdische Pflanzenteile befallen und die Pflanzen vom Boden her angreifen, sowie gegen samenübertragbare Krankheitserreger. Eine besonders gute Wirksamkeit entfalten sie gegen parasitäre Pilze auf oberirdischen Pflanzenteilen.

Als Pflanzenschutzmittel können die erfindungsgemäßen Wirkstoffe mit besonders gutem Erfolg zur Bekämpfung von Venturia-Arten, wie z.B. gegen den Erreger des Apfelschorfs (Fusicladium dendriticum), von Podosphaera-Arten, wie z.B. de Erreger des echten Apfelmehltaus (Podosphaera leucotricha) sowie von Getreidekrankheiten, wie Getreidemehltau und Gerstenmehltau, verwendet werden.

Außerdem sei auf die teilweise systemische Wirkung der Stoffe hingewiesen. So gelingt es, Pflanzen gegen Pilzbefall zu schützen, wenn man de Wirkstoff über den Boden und die Wurzel den oberirdischen Teilen der Pflanzen zuführt.

Als Saatgutbeizmittel angewandt sind die erfindungsgemäßen Verbindungen gegen samenübertragbare pilzliche Pflanzenkrankheiten wirksam und zwar durch Saatgutoberflächendesinfektion, z.B. gegen die Streifenkrankheit der Gerste, als auch systemisch gegen pilzliche Krankheitserreger im Inneren des Saatgutes, wie bei den Flugbränden des Weizens und der Gerste. Außerdem wird durch Saatgutbeizung eine systemische Schutzwirkung gegen pilzliche Infektionen des Sprosses, z.B. gegen Mehltau erreicht.

Als Pflanzenschutzmittel können die erfindungsgemäßen Wirkstoffe zur Saatgut- oder Bodenbehandlung und zur Behandlung oberirdischer Pflanzenteile benutzt werden.

Die Wirkstoffe können in die üblichen Formulierungen übergeführt werden, wie Lösungen, emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Progan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylaryl-polyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen in Mischung mit anderen bekannten Wirkstoffen vorliegen, wie Fungizide, Insektiziden, Akariziden, Nematiziden, Herbiziden, Schutzstoffen gegen Vogelfraß, Wuchsstoffen, Pflanzennährstoffen und Bodenstrukturverbesserrungsmitteln.

Die Wirkstoffe können als solche, in Form ihrer Formulierungen oder der daraus durch weiteres Verdünnen bereiteten Anwendungsformen, wie gebrauchsfertigen Lösungen, Emulsionen, Suspensionen, Pulver, Pasten und Granulate angewendet werden. Die Andwendung geschieht in üblicher Weise, z.B. durch Gießen, Spritzen, Sprühen, Stäuben, Streuen, Trockenbeizen, Feuchtbeizen, Naßbeizen, Schlämmbeizen oder Inkrustieren.

Bei der Verwendung als Blattfungizide können die Wirkstoffkonzentrationen in den Anwendungsformen in einem größeren Bereich variiert werden. Sie liegen im allgemeinen zwischen 0,1 und 0,00001 Gewichtsprozent. Vorzugsweise zwischen 0,05 und 0,0001 %.

Bei der Saatgutbehandlung werden im allgemeinen Wirkstoffmengen von 0,001 bis 50g je Kilogramm Saatgut, vorzugsweise 0,01 bis 10 g benötigt.

Zur Bodenbehandlung sind Wirkstoffmengen von 1 bis 1000 g je cbm Boden, wie insbesondere 10 bis 200 g, erforderlich.

Die vielseitigen Verwendungsmöglichkeiten gehen aus den nachfolgenden Beispielen hervor.

## Beispiel A

Podosphaera-Test (Apfel)/Protektiv

Lösungsmittel: 4,7 Gewichtsteile Aceton

Emulgator: 0,3 Gewichtsteile Alkyl-aryl-polyglykoläther

Waser: 95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der Angegebenen Menge Wasser, welches die genannten Zusätze enthält.

Mit der Spritzflüssigkeit bespritzt man junge Apfelsämlinge, die sich im 4- bis 6-Blattstadium befinden, bis zur Tropfnässe. Die Pflanzen verbleiben 24 Stunden bei 20°C und einer relativen Luftfeuchtigkeit von 70% im Gewächshaus. Anschließend werden sie durch Bestäuben mit Konidien des Apfelmehltauerregers (Podosphaera leucotricha) inokuliert und in ein Gewächshaus mit einer Temperature von 21 bis 23°C und einer relativen Luftfeuchtigkeit von ca. 70% gebracht.

10 Tage nach der Inokulation wird der Befall der Sämlinge bestimmt. Die erhaltenen Boniturwerte werden in Prozent Befall umgerechnet. 0% bedeutet keinen Befall, 100% bedeutet, daß die Pflanzen vollständig befallen sind.

Wirkstoffe, Wirkstoffkonzentrationen und Ergebnisse werden ermittelt. In diesem Test zeigen z.B. folgende Verbindungen eine sehr gute Wirkung, die derjenigen der aus dem Stand der Technik bekannten Verbindungen deutlich überlegen ist: Verbindungen gemäß Herstellungsbeispielen: 2, 3, 1.

## Beispiel B

Fusicladium-Test (Apfel)/Protektiv

Lösungsmittel: 4,7 Gewichtsteile Aceton

Emulgator 0,3 Gewichtsteile Alkyl-aryl-polyglykoläther

Waser: 95,0 Gewichtsteile

Man vermischt die für die gewünschte Wirkstoffkonzentration in der Spritzflüssigkeit nötigen Wirkstoffmengen mit der angegebenen Menge des Lösungsmittels und verdünnt das Konzentrat mit der Angegebenen Menge Wasser, welches die genannten Zusätze enthält.

Mit der Spritzflüssigkeit bespritzt man junge Apfelsämlinge, die sich im 4- bis 6-Blattstadium befinden, bis zur Tropfnässe. Die Pflanzen verbleiben 24 Stunden bei 20°C und einer relativen Luftfeuchtigkeit von 70% im Gewächshaus. Anschließend werden sie mit einer wäßrigen Konidiensuspension des Apfelschorferregers (Fusicladium dendriticum) inokuliert und 18 Stunden lang in einer Feuchtkammer bei 18 bis 20°C und 100% relativer Luftfeuchtigkeit inkubiert.

Die Pflanzen kommen dann erneut für 14 Tage ins Gewächshaus.

15 Tage nach der Inokulation wird der Befall der Sämlinge bestimmt. Die erhaltenen Boniturwerte werden in Prozent Befall umgerechnet. 0% bedeutet keinen Befall, 100% bedeutet, daß die Pflanzen vollständig befallen sind.

Wirkstoffe, Wirkstoffkonzentrationen und Ergebnisse werden ermittelt. In diesem Test zeigen z.B., folgende Verbindungen eine sehr gute Wirkung, die derjenigen der aus dem Stand der Technik bekannten Verbindungen deutlich überlegen ist:
Verbindungen gemäß Herstellungsbeispielen 3, 1.

Beispiel C

Sproßbehandlungs-Test/Getreidemehltau/protektiv
(blattzerstörende Mykose)

Zur Herstellung einer zweckmäßigen Wirkstoffzubereitung nimmt man 0,25 Gew.-Teile Wirkstoff in 25 Gewichsteilen Dimethylformamid und 0,06 Gewichtsteilen Alkylaryl-polyglykoläther auf und gibt 975 Gewichtsteilen Wasser hinzu. Das Konzentrat verdünnt man mit Wasser auf die gewünschte Endkonzentration der Spritzbrühe.

Zur Prüfung auf protektive Wirksamkeit besprüht man die einblättrige Gerstenjungpflanzen der Sorte Amsel mit der Wirkstoffzubereitung taufeucht. Nach Antrocknen bestäubt man die Gerstenpflanzen mit Sporen von Erysiphe graminis var.hordei.

Nach 6 Tagen Verweilzeit der Pflanzen bei einer Temperatur von 21—22°C und einer Luftfeuchtigkeit von 80—90% wertet man den Besatz mit Mehltaupustein aus. Der Befallsgrad wird in Prozenten des Befalls der unbehandelten Kontrollpflanzen ausgedrückt. Dabei bedeutet 0% keinen Befall und 100% den gleichen Befallsgrad wie bei der unbehandelten Kontrolle. Der Wirkstoff ist umso wirksamer, je geringer der Mehltaubefall ist.

Wirkstoffe, Wirkstoffkonzentration in der Spritzbrühe und Befallsgrade werden ermittelt. In diesem Test zeigen z.B. folgende Verbindungen eine sehr gute Wirkung, die derjenigen der aus dem Stand der Technik bekannten Verbindungen überlegen ist:

Verbindungen gemäß Herstellungsbeispielen 2, 3, 1, 4.

Beispiel D

Gerstenmehltau-Test (Erysiphe graminis var. hordei): systemisch
(pilzliche Getreidesproßkrankheit)

Die Anwendung der Wirkstoffe erfolgt als pulverförmige Saatgutbehandlungsmittel. Sie werden hergestellt durch Abstrecken des jeweiligen Wirkstoffes mit einem Gemisch aus gleichen Gewichtsteilen Talkum und Kieselgur zu einer feinpulverigen Mischung mit der gewünschten Wirkstoffkonzentration.

Zur Saatgutbehandlung schüttelt man Gerstensaatgut mit dem abgestreckten Wirkstoff in einer verschlossenen Glasflasche. Das Saatgut sät man mit 3 x 12 Korn in Blumentöpfe 2 cm tief in ein Gemisch aus einem Volumteil Frühstorfer Einheitserde und einem Volumteil Quarzsand ein. Die Keimung und der Auflauf erfolgen unter günstigen Bedingungen im Gewächsbaus. 7 Tage nach der Aussaat, wenn die Gerstenpflanzen ihr erstes Blatt entfaltet haben, werden sie mit frischen Sporen von Erysiphe graminis var. hordei bestäubt und bei 21—22°C und 80—90% rel. Luftfeuchte und 16-stündiger Belichtung weiter Kultiviert. Innerhalb von 6 Tagen bilden sich an den Blättern die typischen Mehltaupusteln aus.

Der Befallsgrad wird in Prozenten des Befalls der unbehandelten Kontrollpflanzen ausgedrückt. So bedeutet 0% keinen Befall und 100% den gleichen Befallsgrad wie bei der unbehandelten Kontrolle. Der Wirkstoff ist um so wirksamer je geringer der Mehltaubefall ist.

Wirkstoffe, Wirkstoffkonzentrationen im Saatgutbehandlungsmittel sowie dessen Aufwandmenge und der prozentuale Mehltaubefall werden ermittelt. Bei diesem Test zeigen z.B. folgende Verbindungen eine sehr gute Wirkung, die derjenigen der aus dem Stand der Technik bekannten Verbindungen deutlich überlegen ist:

Verbindungen gemäß Herstellungsbeispielen: 3, 4.

Beispiel E

Saatgutbeizmittel-Test/Streifenkrankheit der Gerste
(samenbürtige Mykose)

Zur Herstellung eines zweckmäßigen Trockenbeizmittels verstreckt man den Wirkstoff mit einem Gemisch aus gleichen Gewichtsteilen Talkum und Kieselgur zu einer feinpulverigen Mischung mit der Gewünschten Wirkstoffkonzentration.

Zur Beizung schüttelt man Gerstensaatgut, das durch Drehslera graminea (Syn. Helminthosporin gramineum) natürlich verseucht ist, mit dem Beizmittel in einer verschlossenen Glasflasche. Das Saatgut setzt man auf feuchten Filterscheiben in verschlossenen Petrischalen im Kühlschrank 10 Tage lang einer Temperatur von 4°C aus. Dabei wird die Keimung der Gerste und gegebenenfalls auch der Pilzsporen eingeleitet.

Anschließend sät man die vorgekeimte Gerste mit 2 x 50 Korn 2 cm tief in Fruhstorfer Einheitserde und kultivert sie im Gewächshaus bei Temperaturen um 18°C in Saatkästen, die täglich 16 Stunden dem Licht ausgesetzt werden. Innerhalb von 3 bis 4 Wochen bilden sich die typischen Symptome der Streifenkrankheit aus.

Nach dieser Zeit bestimmt man die Anzahl der kranken Pflanzen in Prozent der insgesamt aufgelaufenen Pflanzen. Der Wirkstoff ist umso wirksamer je weniger Pflanzen erkrankt sind.

Wirkstoffe, Wirkstoffkonzentrationen im Beizmittel, Beizmittelaufwandmengen und Anzahl der erkrankten Pflanzen werden ermittelt. In diesem Test zeigen z.B. folgende Verbindungen eine sehr gute Wirkung, die derjenigen der aus dem Stand der Technik bekannten Verbindungen deutlich überlegen ist:

Verbindungen gemäß Herstellungsbeispiel 3.

## Herstellungsbeispiele

### Beispiel 1

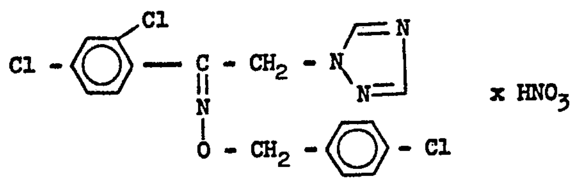

13,8 g (0,05 Mol) 1 - (2,4 - Dichlorphenyl) - 1 - oximino - 2 - (1,2,4 - triazol - 1 - yl) - äthan werden zu einer Mischung aus 100 ml konzentrierter Natronlauge (45%ig), 100 ml Toluol und 3 g Tetrabutyl-ammoniumjodid zugegeben und mit 25 g (0,15 Mol) 4-Chlorbenzylchlorid 20 Stunden bei Raumtemperatur verrührt. Danach wird die organische Phase abgetrennt, zweimal mit je 100 ml gesättigter Natriumchloridlösung gewaschen, über Natriumsulfat getrocknet und im Vakuum durch Abdestillieren des Lösungsmittels eingeengt. Der Rückstand wird in 100 ml n-Butanol gelöst und tropfenweise bei Raumtemperatur mit 3,2 g konzentrierter Salpetersäure (98 %-ig) versetzt. Man läßt 15 Stunden bei 0°C stehen und saugt den ausgefallenen kristallinen Niederschlag ab. Man erhält 7 g (30,5% der Theorie) 1 - (2,4 - Dichlorphenyl) - 1 - (4 - chlorbenzyloximino) - 2 - (1,2,4 - triazol - 1 - yl) - äthan - nitrat vom Schmelzpunkt 130—135°C (Zers.).

### Herstellung der Vorstufen

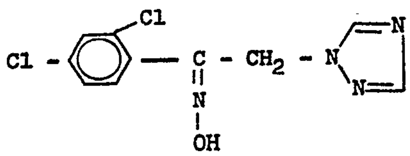

106,8g (0,44 Mol) 1 - (2,4 - Dichlorphenyl) - 2 - (1,2,4 - triazol - 1 - yl) - äthan - 1 - on werden in 780 ml Aethanol gelöst, mit 48 g Hydroxylammoniumhydrochlorid versetzt und 5 Stunden unter Rückfluß erhitzt. Danach wird das Reaktionsgemisch mit 1000 ml Wasser versetzt und filtriert. Man erhält 51 g (45% der Theorie) 1 - (2,4 - Dichlorphenyl) - 1 - oximino - 2 - (1,2,4 - triazol - 1 - yl) - äthan vom Schmelzpunkt 165—170°C.

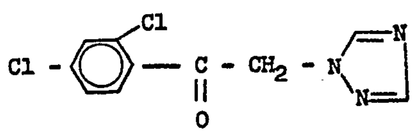

269 g (1 Mol) $\omega$-Brom-2,4-dichloracetophenon werden in 250 ml Acetonitril gelöst. Diese Lösung tropft man zu einer unter Rückfluß siedenden Suspension von 69 g (1 Mol) 1,2,4-Triazol und 150 g Kaliumcarbonat in 2 l Acetonitril. Nach 20-stündigem Erhitzen unter Rückfluß wird die erkaltete Suspension filtriert, das Filtrat vom Lösungsmittel befreit und der Rückstand mit Essigester aufgenommen, mit Wasser gewaschen, über Natriumsulfat getrocknet und vom Lösungsmittel befreit. Der Essigester-Rückstand kristallisiert beim Versetzen mit Isopropanol aus. Nach dem Umkristallisieren aus Ligroin/Isopropanol erhält man 154 g (60% der Theorie) $\omega$-(1,2,4-Triazol-1-yl)-2,4-dichloracetophenon vom Schmelzpunkt 117°C.

In entsprechender Weise werden die folgenden Verbindungen der allgemeinen Formel

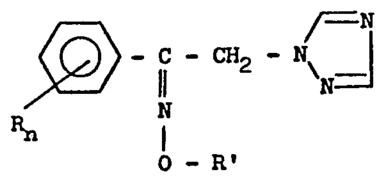

erhalten:

| Bsp. Nr. | $R_n$ | R' | Schmelzpunkt (°C) |
|---|---|---|---|
| 2 | 2,4-$Cl_2$ | 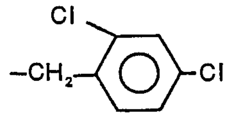 | 135–37 (Zers.) (x $HNO_3$) |
| 3 | 2,4-$Cl_2$ | $C_2H_5$ | 145–46 (Zers.) (x HCl) |
| 4 | 2,4-$Cl_2$ | $C_3H_7$ | 108–12 |
| 5 | 2,4-$Cl_2$ | $C_2H_5$ | 65–72 |
| 6 | 2,4-$Cl_2$ | 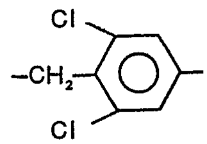 | 160 (Zers.) (x $HNO_3$) |
| 7 | 2,4-$Cl_2$ | $CH_3$ | 90–92 (x $HNO_3$) |
| 8 | 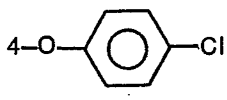 | $C_3H_7$ | 154–156 (x $HNO_3$) |
| 9 | 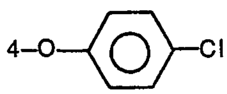 | $C_4H_9$ | 54–55 |
| 10 | 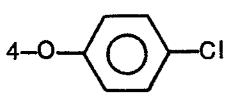 | 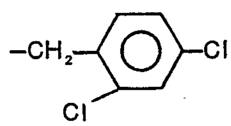 | 110–118 |
| 11 | 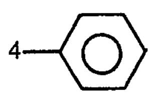 | 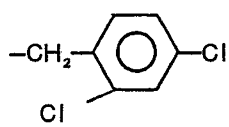 | 129–130 |
| 12 | 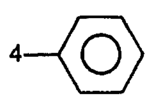 | $C_4H_9$ | 129–130 |
| 13 | 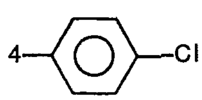 | 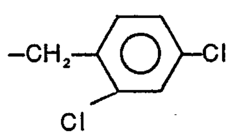 | 166 |
| 14 | 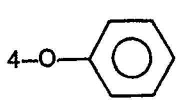 | $C_4H_9$ | 142–144 (x $HNO_3$) |
| 15 | 2,5-$Cl_2$ | 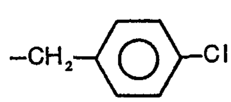 | 158–160 (x HCl) |
| 16 | 3,4-$Cl_2$ | 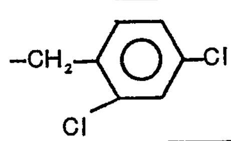 | 120–121 |

Weiterhin können noch die nachfolgend aufgeführten Verbindungen der allgemeinen Formel (I) in ähnlicher Weise Synthetisiert werden:

| $R_n$ | R' |
|---|---|
| 2,4-$Cl_2$ | $-CH_2-CH=CH_2$ |
| 2,4-$Cl_2$ | 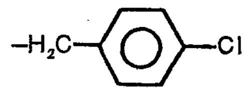 |
| 2,4-$Cl_2$ | 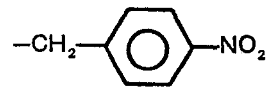 |
| 2,4-$Cl_2$ | $-CH_2-C\equiv CH$ |
| 3,4-$Cl_2$ | $-CH_2-CH=CH_2$ |
| 3,4-$Cl_2$ | $-C_4H_9$ |
| 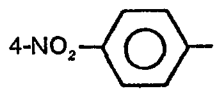 | $-CH_2-CH=CH_2$ |
| 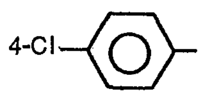 | $-C_4H_9$ |
| 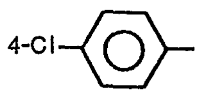 | $-C_2H_5$ |
| 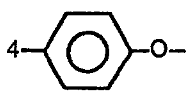 | 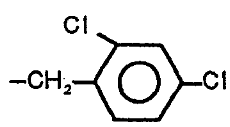 |
| 4-Br | 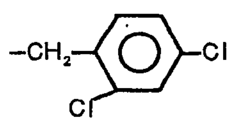 |

**Patentansprüche**

1. Oximino-triazolyl-äthane der allgemeinen Formel

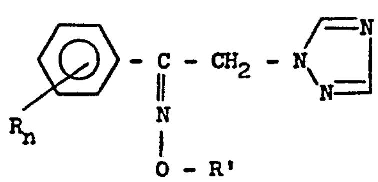

(I)

in welcher

R für Halogen, Nitro und Cyano, für alkyl und alkylsulfonyl mit jeweils 1 bis 4 Kohlenstoffatomen, für

Alkoxy und Alkylthio mit 1 bis 2 Kohlenstoffatomen, für Halogenalkyl mit bis zu 4 Kohlenstoffund bis zu 5 Halogenatomen, ferner für Phenyl oder Phenoxy steht, wobei letztere beiden Reste durch Halogen, Cyano, Nitro, Halogenalkyl mit bis zu 2 Kohlenstoff- und bis zu 3 Halogenatomen substituiert sein können,

R' für Alkyl, Alkenyl und Alkinyl mit jeweils bis zu 4 Kohlenstoffatomen steht, sowie für Benzyl oder Styryl steht, wobei die beiden letztgenannten Reste durch Halogen, Cyano, Nitro, Amino, Alkyl mit bis zu 4 Kohlenstoffatomen, Phenyl, Phenoxy und Halogenalkyl mit bis zu 2 Kohlenstoff- und bis zu 3 Halogenatomen substituiert sein können, und

n für ganze Zahlen von 0 bis 3 steht,

und deren physiologisch verträglichen Säureadditions- Salze und Metallsalz-Komplexe.

2. Verfahren zur Herstellung von Oximino-triazolyl-äthanen, dadurch gekennzeichnet, daß man Salze der Oxime der Formel

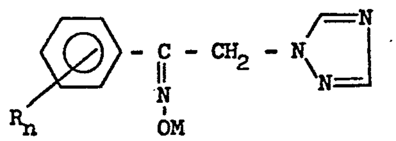

(II)

in welcher

R und n die in Anspruch 1 angegebene Bedeutung haben, und

M für ein Alkalimetall, eine quarternäre Ammonium- oder Phosphonium-Gruppe steht,

mit einem Halogenid der Formel

R'—Hal (III)

in welcher

R' die in Anspruch 1 angegebene Bedeutung hat und

Hal für Chlor oder Brom steht,

in Gegenwart eines Verdünnungsmittels umsetzt, und gegebenenfalls noch die erhaltenen Verbindungen durch Umsetzungen mit säuren in die Salze bzw. durch Reaktion mit Metallsalzen in die entsprechenden Metallkomplexe überführt.

3. Fungizide Mittel, gekennzeichnet durch einen Gehalt an mindestens einem Oximino-triazolyläthan gemäß Anspruch 1.

4. Verwendung von Ominino-triazolyl-äthanen gemäß Anspruch 1 zur Bekämpfung von Pilzen.

**Revendications**

1. Oximino-triazolyl-éthanes répondant à la formule générale:

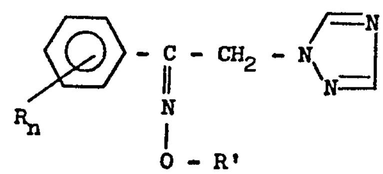

(I)

dans laquelle

R représente un atome d'halogène, un groupe nitro ou un groupe cyano, un groupe alkyle ou un groupe alkylsulfonyle contenant chacun 1 à 4 atomes de carbone, un groupe alcoxy ou un groupe alkylthio contenant 1 ou 2 atomes de carbone, un groupe halogénalkyle contenant jusqu'à 4 atomes de carbone et jusqu'à 5 atomes d'halogènes, de même qu'un groupe phényle ou un groupe phénoxy, ces deux derniers groupes pouvant être substitués par un atome d'halogène, un groupe cyano, un groupe nitro ou un groupe halgénalkyle contenant jusqu'à 2 atomes de carbone et jusqu'à 3 atomes d'halogènes,

R' représente un groupe alkyle, un groupe alcényle, ou un groupe alcinyle contenant chacun jusqu'à 4 atomes de carbone, de même qu'un groupe benzyle, ou un groupe styryle, ces deux derniers groupes pouvant être substitués par un atome d'halogène, un groupe cyano, un groupe nitro, un groupe amino, un groupe alkyle contenant jusqu'à 4 atomes de carbone, un groupe phényle, un groupe phénoxy ou un groupe halogénealkyle contenant jusqu'à 2 atomes de carbone et jusqu'à 3 atomes d'hydrogènes, et

n représente un nombre entier de 0 à 3,

ainsi que leurs complexes de sels métalliques et leurs sels d'addition d'acide physiologiquement compatibles.

2. Procédé de préparation d'oximino-triazolyl-éthanes, caractérisé en ce qu'on fait réagir des sels des oximes de formule:

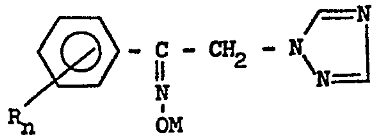

(II)

dans laquelle
R et n ont les significations indiquées dans la revendication 1, et
M représente un métal alcalin, un groupe phosphonium ou ammonium quaternaire,
avec un halogénure de formule:

R'—Hal (III)

dans laquelle
R' a la signification indiquée dans la revendication 1, et
Hal représente un atome de chlore ou un atome de brome, en présence d'un diluant, et l'on transforme encore éventuellement les composés obtenus en sels par des réactions avec des acides, ou encoure on les transforme en complexes métalliques correspondants par réaction avec des sels métalliques.

3. Agent fongicide, caractérisé en ce qu'il contient au moins un oximino-triazolyl-éthane suivant la revendication 1.

4. Utilisation d'oximino-triazolyl-éthanes suivant la revendication 1 pour combattre les champignons.

**Claims**

1. Oximino-triazolyl-ethanes of the general formula

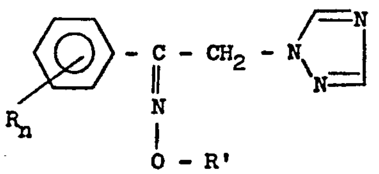

(I)

in which
R represents halogen, nitro and cyano, alkyl and alkylsulphonyl with in each case 1 to 4 carbon atoms; alkoxy and alkylthio with 1 to 2 carbon atoms; halogenoalkyl with up to 4 carbon atoms and up to 5 halogen atoms; furthermore represents phenyl or phenoxy, it being possible for the latter two radicals to be substituted by halogen, cyano, nitro, halogenoalkyl with up to 2 carbon atoms and up to 3 halogen atoms,
R' represents alkyl, alkenyl and alkinyl with in each case up to 4 carbon atoms; and benzyl or styryl, it being possible for the two last-mentioned radicals to be substituted by halogen, cyano, nitro, amino, alkyl with up to 4 carbon atoms, phenyl, phenoxy and halogenoalkyl with up to 2 carbon atoms and up to 3 halogen atoms and
n represents integers from 0 to 3,
and their physiologically acceptable acid addition salts and metal salt complexes.

2. Process for the preparation of oximino-triazolylethanes, characterized in that salts of oximes of the formula

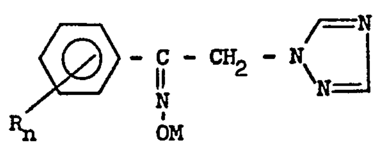

(II)

in which
R and n have the meaning indicated in claim 1 and M represents an alkali metal or a quaternary ammonium or phosphonium group, are reacted with a halide of the formula

R'—Hal (III)

in which

R' has the meaning indicated in claim 1 and

Hal represents chlorine or bromine, in the presence of a diluent, and if appropriate the resulting compounds are also converted into the salts by reactions with acid or into the corresponding metal complexes by reaction with metal salts.

3. Fungicidal agents, characterised in that they contain at least one oximino-triazolyl-ethane according to Claim 1.

4. Use of oximino-triazolyl-ethanes according to Claim 1 for combating fungi.